Europäisches Patentamt

(19) European Patent Office

Office européen des brevets

(11) Publication number: **0 114 619**
**B1**

(12) **EUROPEAN PATENT SPECIFICATION**

(45) Date of publication of patent specification: **02.08.89**

(51) Int. Cl.⁴: **B 01 D 1/14, A 61 L 2/20**

(21) Application number: **84100287.6**

(22) Date of filing: **12.01.84**

(54) **A method and an arrangement for the volatilization of a liquid.**

(30) Priority: **25.01.83 SE 8300356**

(43) Date of publication of application:
**01.08.84 Bulletin 84/31**

(45) Publication of the grant of the patent:
**02.08.89 Bulletin 89/31**

(84) Designated Contracting States:
**AT BE CH DE FR GB IT LI LU NL SE**

(56) References cited:
**DE-B-2 744 637**
**DE-C- 301 781**
**DE-C- 404 480**
**US-A-2 817 415**

(73) Proprietor: **AB TETRA PAK**
**Box 61**
**S-221 00 Lund (SE)**

(72) Inventor: **Hilmersson, Anders**
**Eksjögatan 11**
**S-252 51 Helsingborg (SE)**
Inventor: **Lagerstedt, Jan**
**Balladgatan 49**
**S-214 71 Malmö (SE)**
Inventor: **Andersson, Helge**
**Karhögstorg 4B**
**S-223 55 Lund (SE)**

(74) Representative: **Müller, Hans-Jürgen, Dipl.-Ing. et al**
**Müller, Schupfner & Gauger Lucile-Grahn-Strasse 38 Postfach 80 13 69**
**D-8000 München 80 (DE)**

Courier Press, Leamington Spa, England.

## Description

The invention concerns a method for volatilizing a liquid through blending with heated gas within a chamber according to the opening part of claim 1 and an apparatus for volatilizing a liquid by heated gas according to the opening part of the apparatus claim 6.

The manufacture of packing containers for products with long keeping quality e.g. sterilized juices or the like takes place in packaging machines of the aseptic type. In the packaging machine a material web or material blanks are converted to packing containers which are filled with sterile contents and sealed. The manufacture has to take place under aseptic conditions and the packaging machine thus must be sterilized internally before production commences. This is done preferably by blending a liquid sterilized agent with hot sterile air so that a gas saturated with sterilized agent of a suitable temperature and dew point is obtained. The gas is conducted around the inside of the packaging machine, the sterilizing agent condensing on the relatively cool parts of the machine which come into contact with the gas. As a result a thin uniform layer of sterilizing agent is formed which sterilizes the surface with which the contents or the packing material later will come into contact. Before the putting into operation of the packaging machine the sterilizing agent is removed by hot air which is conducted around the machine so that the sterilizing agent is evaporated again and can be discharged together with the air. In this manner the inside of the machine is sterilized and the production of aseptic packages can commence.

The sterilization method described has been found to work well in practice under the condition that the dew point of the gas mixture lies sufficiently high in relation to the temperature of the machine parts which are to be sterilized. However, if the machine parts which are to be sterilized are too high a temperature at the start of the presterilization, condensation will not take place on the surface of the parts and the sterilization consequently will be incomplete. This will happen e.g. in machines which are located in abnormally warm localities and in regions with a warm climate. The mixture of sterilizing agent and air which has been used up to now was produced through injection of liquid sterilizing agent into a pipe through which passed a flow of hot sterile air. The dew point of the mixture obtained in this manner will attain maximum 30-40°C which in certain cases proves to be insufficient. It has not been possible, however, up to now to obtain by means of the prior art a higher dew point with the large flows which are necessary for the production of sufficient gas quantities.

A method according to the opening part of claim 1 is described in DE-C-301 781. According to said method a jet of droplets is introduced into the chamber from below so that the stream of the droplets within the chamber is substantially the same as the upward moving direction of the gas or stream.

It is the objective of the present invention to increase the dew point of the mixture of gas and volatilized liquid droplet with a greater volume per unit of time.

The invention is claimed in claim 1.

According to the invention the jet of droplets is injected into the chamber from the upper side thereof in a substantially counter-current direction (opposite direction) with regard to the stream of the heated gas and said stream of gas is guided by guide rails arranged in a helical shape along the inner surface of the wall of the chamber leaving the center free for the substantially conical jet of droplets.

A droplet size being introduced in counter-current direction of between 10-80 μm is preferred.

Further embodiments of the invention are claimed in the sub-claims.

Furthermore, an apparatus according to the opening part of claim 6 is described in US-A-2 817 415 describing an angle of inclination of about 45°. The inner diameter of the helical guide rail is constant and there is a small gap between the inner edge of the helical guide rail and the outer surface of the tube.

In contrary to this reference the invention according to claim 6 does not comprise such angle of inclination. In the contrary, the invention facilitates to hinder water droplets to reach the lower end because the liquid inlet means is a spray nozzle for introducing a substantially conical jet of droplets of said liquid in the prolongation of the geometrical center axis of said helical guide rail. The inner diameter thereof forms a center opening around said center axis widening in the direction away from said spray nozzle.

The water droplets can contact the cylindrical wall which is very well heated by the heated gas in order to reach the very high dew point and a big rate of volatilized liquid. While the smaller droplets immediately volatilized mostly in the upper part of the chamber the bigger droplets reach the heated cylindrical wall of the chamber because the helical guide rail is mainly radial without any angle of inclination although the jet of droplets is not substantially hindered by guide rails to torn the film of liquid into very small droplets.

A further embodiment is claimed in claim 9.

As a result the dew point of the mixture of gas and volatilized liquid droplet is very much increased with a greater volume per unit of time. The present invention is suitable for the application in the presterilization of packaging machines of the aseptic type. According to the present invention it is possible to rapidly and completely blend and volatilize the liquid in the hot gas. As a result thereof a mixture of a dew point of up to over 60° C can be produced very rapidly (over 100 m³/h). A high production capacity can be reached. Furthermore, the present apparatus does not comprise movable parts and is very simple in its design which entails low manufacturing and low operating costs.

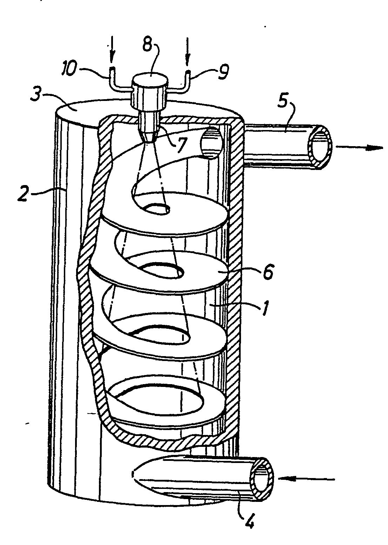

A preferred embodiment of the method and of the apparatus in accordance with the invention will now be described in greater detail with special reference to the enclosed drawing which shows schematically an apparatus in accordance with the invention. The apparatus is partly cut open in order to show its interior design, and only the parts essential for an understanding of the invention have been included.

The apparatus in accordance with the invention comprises a cylindrical blending chamber 1 which is surrounded by a casing with a cylindrical shell wall 2 and end walls 3. The apparatus further comprises feed and discharge lines 4, 5 respectively which are in the form of pipes joining the cylindrical shell 2 tangentially. The feed line 4 is located near the lower end wall of the blending chamber and the discharge line 5 is located near the upper end wall 3 of the blending chamber 1. The cylindrical shell wall 2 as well as the end walls 3 and the lines 4, 5 are made of stainless steel, the shell wall 2, for reasons which will be explained in more detail in the following, having a relatively great thickness of approx. 5 mm. The total height of the container is approx. 0.5 m and the diameter of the chamber is 150 mm.

Inside the chamber 1 there is a helical guide rail 6 which runs along the shell surface between the two end walls of the blending chamber. The guide rail 6 is made up from a striplike plate segment coiled in the form of a helix whose width diminishes successively from approx. 60 mm at the upper end of the blending chamber to approx. 20 mm at the lower end of the blending chamber. Since the guide rail 6 rests with its one edge against the inner surface of the shell wall 2, the successively diminishing width means that the guide rail has a centrally located opening, widening conically, whose cone angle is approx. 15°.

As is also evident from the drawing the blending chamber 1 is provided at its upper end with a nozzle 7 which is arranged in the centre of the end wall 3. The nozzle 7 opens out into the blending chamber 1 immediately below the inner surface of the end wall 3 and its central location means that the outlet opening of the nozzle will be positioned in the prolongation of the geometric centre axis which is common to the guide rail 6 in the form of a helix as well as to the cylindrical shell wall 2 of the blending chamber 1. The nozzle 7 is a spray nozzle which generates drops in the order of magnitude of 10-80µm which are spread in a substantially conical jet whose cone angle is approx. 15° and thus corresponds to the cone angle of the centre opening of the guide rail 6. Outside the blending chamber the nozzle 7 is connected via a valve to the feed lines 9, 10 for liquid and gas respectively. The feed line 9 is connected to a liquid tank (not shown) and the feed line 10 is connected to a source of sterile air.

When the apparatus in accordance with the invention is placed into a packaging machine of known type, e.g. a packaging machine of the type which is described in the US patent no. 3 911 642, the feed and discharge lines are connected to the machine's system of sterile air and sterilizing liquid respectively.

The sterilizing liquid usually consists of 35% hydrogen peroxide and the sterile air is the ambient atmosphere which has been made to pass a sterile filter and has been heated to a temperature of approx. 300°C. When presterilization is to be initiated the hot sterile air is introduced via the feed line 4 at a rate of approx. 150 m/s. Since the feed line 4 is arranged, as mentioned previously, tangentially in relation to the shell wall 2, a circulating movement will be imparted to the air. With the help of the guide rail 6 the circulating air is induced to rise upwards in the blending chamber 1 gradually to reach the discharge line 5 likewise joining, tangentially. After the hot air has been allowed to pass for some time through the blending chamber 1 and the latter has been heated to substantially the same temperature as the air, that is to say 300°C, hydrogen peroxide and sterile air are fed at high pressure via lines 9 and 10 respectively to the nozzle 7. Sterilizing liquid is injected via the outlet opening of the nozzle into the blending chamber 1 in countercurrent direction in relation to the air flow, that is to say downwards. The nozzle finely divides the liquid so that it is injected in the form of drops of a size of 10-80µm. The nozzle 7 sprays the liquid in a substantially evenly distributed conical jet whose acute angle is approx. 15°. Since a circulating movement mainly along the walls of the blending chamber 1 has been imparted to the gas or hot air introduced into the blending chamber 1, the sterilizing liquid fed via the nozzle 7 will be introduced into the centre of the circulating movement and more particularly along the centre axis of the helical movement of the gas. The smaller drops of sterilizing liquid issuing from the nozzle 7 will be blended very rapidly with the rotating hot air and be volatilized, whilst the medium-sized and larger drops will be spurted farther down into the blending chamber 1 before being heated to such a degree that they are volatilized. Owing to the countercurrent principle the largest drops, when they approach the lower end of the blending chamber 1, will encounter hot air of the highest temperature, which ensures that a satisfactory volatilization will take place even of relatively larger drops of sterilizing agent. The helical movement of the hot air means that the drops when they are captured by the hot air are entrained out whilst being volatilized towards the periphery of the blending chamber where, if they have not achieved volatilization beforehand, they encounter the cylindrical shell wall 2 of the blending chamber 1 which has been heated by hot air to a high temperature and consequently causes immediate volatilization of any remaining drops.

The injection of the sterilizing liquid into the blending chamber preferably takes place intermittently at such intervals that an air saturated with sterilizing agent of an appropriate dew point is obtained. In the intervals between the injections the cylindrical shell wall 2, owing to its great material thickness, can absorb and store thermal energy from the hot air passing. This thermal energy is utilized insubsequent injections of sterilizing liquid and has been found to make it possible to raise the dew point of the mixture still further by approx. 10°.

The successive volatilization and the vigorous blending and centrifugal effect at the injection of hot air into the blending chamber has proved in practice to achieve a very effective volatilization and as a result a very high dew point of the finished mixture can be maintained. Measurements show that under optimum conditions the dew point lies only one or two degrees below the theoretical upper limit which is defined by the heat content of the air (practically 60°C). The helical guide rail serves not only for conducting gas from the inlet to the outlet in such a manner that it maintains volatilization in the most effective manner possible, but also for strongly reducing the noise level during operation.

**Claims**

1. Method for volatilizing a liquid through blending with heated gas within a chamber (1), wherein the heated gas moves quickly and upwardly in said chamber in a substantially helical movement and wherein the liquid is introduced in the form of a substantially conical jet of droplets into the centre of the helical movement, characterized in that the jet of droplets is injected into said chamber from the upper side thereof in a substantially countercurrent direction with regard to the stream of the heated gas and that said stream of gas is guided by guide rails (6) arranged in a helical shape along the inner surface of the wall (2) of the chamber (1) leaving the center free for the substantially conical jet of droplets.

2. Method as claimed in claim 1, characterized in that the droplets are introduced in countercurrent direction in a size of 10-80 µm.

3. Method as claimed in claims 1 or 2, characterized in that heated air of a temperature of approx. 300° C is introduced at a rate of flow of approx. 150 m/s.

4. Method as claimed in anyone of the preceding claims, characterized in that droplets of hydrogen peroxide are introduced by the help of sterile air.

5. Method as claimed in anyone of the preceding claims, characterized in that the jet of droplets is intermittently introduced.

6. Apparatus for volatilizing a liquid by heated gas comprising a chamber (1) with an inlet opening (4) at the lower end and an outlet opening (5) at the upper end for introducing said heated gas and for feeding out said gas with volatilized liquid, wherein the chamber (1) is provided, at the inner surface of its substantially cylindrical wall (2), with at least one helical guide rail (6), and an inlet means for said liquid at the upper end of the chamber (1), characterized in that said liquid inlet means is a spray nozzle (7) for introducing a substantially conical jet of droplets of said liquid in the prolongation of the geometrical centre axis of said helical guide rail (6), the inner diameter of which forms a centre opening, around said centre axis, widening in the direction away from said spray nozzle (7).

7. Apparatus as claimed in claim 6, characterized in that the centre opening of the guide rail (6) has a conical angle which substantially corresponds to the spreading angle of said substantially conical jet of droplets.

8. Apparatus as claimed in claim 6 or 7, characterized in that said helical guide rail (6) is prepared of a strip like plate segment and web, respectively, coiled in the form of a helix whose width diminishes successively from about 6 cm at the upper end of the chamber (1) to about 2 cm at its lower end.

9. Apparatus as claimed in anyone of claims 6 - 8, characterized in that the wall (2) of the substantially cylindrical chamber has a relatively thick wall thickness in order to store thermal energy supplied by the heated gas.

**Patentansprüche**

1. Verfahren zum Verdampfen einer Flüssigkeit durch Vermischen mit Heißgas innerhalb einer Kammer (1), wobei das Heißgas in der Kammer mit einer im wesentlichen schraubenförmigen Bewegung schnell und aufwärts strömt und wobei die Flüssigkeit in form eines im wesentlichen konischen Tröpfchenstrahls in die Mitte der schraubenförmigen Bewegung eingeleitet wird, dadurch gekennzeichnet, daß der Tröpfchenstrahl in die Kammer von deren Oberseite im wesentlichen im Gegenstrom relativ zu dem Heißgasstrom eingepreßt wird, und daß der Gasstrom von Leitschienen (6) geführt wird, die schraubenförmig entlang der Innenfläche der Wand (2) der Kammer (1) angeordnet sind, so daß die Mitte für den im wesentlichen konischen Tröpfchenstrahl frei bleibt.

2. Verfahren nach Anspruch 1, dadurch gekennzeichnet, daß die Tröpfchen in Gegenstromrichtung mit einer Größe von 10-80 µm eingeleitet werden.

3. Verfahren nach Anspruch 1 oder 2, dadurch gekennzeichnet, daß Heißluft mit einer Temperatur von ca. 300 °C mit einer Strömungsgeschwindigkeit von ca. 150 m/s eingeleitet wird.

4. Verfahren nach einem der vorhergehenden Ansprüche, dadurch gekennzeichnet, daß Hydrogenperoxidtröpfchen mit Hilfe steriler Luft eingeleitet werden.

5. Verfahren nach einem der vorhergehenden Ansprüche, dadurch gekennzeichnet, daß der Tröpfchenstrahl intermittierend eingeleitet wird.

6. Einrichtung zum Verdampfen einer Flüssigkeit mittels Heißgas, mit einer Kammer (1), die am Oberende eine Zuführöffnung (4) und am Unterende eine Austragöffnung (5) zur Einleitung des Heißgases bzw. zum Austrag des Gases mit verdampfter Flüssigkeit aufweist, wobei die Kammer (1) an der Innenfläche ihrer im wesentlichen zylindrischen Wand (2) wenigstens eine schraubenförmigen Leitschiene (6) aufweist, und mit einem Flüssigkeits-Einleitorgan am Oberende der Kammer (1), dadurch gekennzeichnet, daß das Flüssigkeits-Einleitorgan eine Zerstäuberdüse (7) ist, die einen im wesentlichen konischen Tröpfchenstrahl der Flüssigkeit in Verlängerung der geometrischen Mittenachse der schraubenförmigen Leitschiene (6) einleitet, wobei der Innendurchmesser der Leitschiene eine Mittenöffnung um diese Mittenachse bildet und die Mittenöffnung sich in der von der Zerstäuberdüse (7) wegführenden Richtung erweitert.

7. Einrichtung nach Anspruch 6, dadurch gekennzeichnet, daß die Mittenöffnung der Leitschiene (6) einen Kegelwinkel aufweist, der im wesentlichen dem Ausbreitungswinkel des im wesentlichen konischen Tröpfchenstrahls entspricht.

8. Einrichtung nach Anspruch 6 oder 7, dadurch gekennzeichnet, daß die schraubenförmige Leitschiene (6) aus einem bandartigen Blechsegment bzw. -steg besteht, der in Form einer Helix gewickelt ist, deren Weite allmählich von ca. 6 cm am Oberende der Kammer (1) auf ca. 2 cm an deren Unterende abnimmt.

9. Einrichtung nach einem der Ansprüche 6-8, dadurch gekennzeichnet, daß die Wand (2) der im wesentlichen zylindrischen Kammer eine relativ große Wandstärke aufweist, um von dem Heißgas zugeführte Wärmeenergie zu speichern.

## Revendications

1. Méthode pour volatiliser un liquide par mélange avec un gaz chauffé dans une chambre (1), dans laquelle le gaz chauffé se déplace rapidement vers le haut dans ladite chambre suivant un mouvement sensiblement hélicoïdal et dans laquelle le liquide est introduit sous la forme d'un jet sensiblement conique de gouttelettes au centre du mouvement hélicoïdal, caractérisée en ce que le jet de gouttelettes est injecté dans ladite chambre par son côté supérieur sensiblement à contre-courant par rapport au flux du gaz chauffé, et en ce que ledit flux de gaz est guidé par des rails de guidage (6) disposés hélicoïdalement le long de la surface intérieure de la paroi (2) de la chambre (1) de manière à laisser le centre libre pour le passage du jet sensiblement conique de gouttelettes.

2. Méthode suivant la revendication 1, caractérisée en ce que les gouttelettes sont introduites à contre-courant et leur dimension est de 10 à 80 µm.

3. Méthode suivant la revendication 1 ou 2, caractérisée en ce que l'air chauffé à une température de 300°C environ est introduit à une vitesse de 150 m/s environ.

4. Méthode suivant l'une quelconque des revendications précédentes, caractérisée en ce que des gouttelettes d'eau oxygénée sont introduites à l'aide d'air stérile.

5. Méthode suivant l'une quelconque des revendications précédentes, caractérisée en ce que le jet de gouttelettes est introduit par intermittences.

6. Dispositif pour volatiliser un liquide par un gaz chauffé, comprenant une chambre (1) qui comporte un orifice d'entrée (4) à l'extrémité inférieure et un orifice de sortie (5) à l'extrémité supérieure, pour introduire ledit gaz chauffé et pour évacuer ledit gaz avec le liquide volatilisé, dans lequel la chambre (1) est pourvue, à la surface intérieure de sa paroi sensiblement cylindrique (2), d'au moins un rail de guidage hélicoïdal (6), et de moyens d'entrée pour ledit liquide à l'extrémité supérieure de la chambre (1), caractérisé en ce que lesdits moyens d'entrée de liquide sont constitués d'une buse de pulvérisation (7) pour introduire un jet sensiblement conique de gouttelettes dudit liquide en prolongement de l'axe central géométrique dudit rail de guidage hélicoïdal (6) dont le diamètre intérieur définit une ouverture centrale, autour dudit axe central, qui s'élargit au fur et à mesure qu'on s'éloigne de ladite buse de pulvérisation (7).

7. Dispositif suivant la revendication 6, caractérisé en ce que l'ouverture centrale du rail de guidage (6) présente un angle de conicité qui correspond sensiblement à l'angle de pulvérisation dudit jet sensiblement conique de gouttelettes.

8. Dispositif suivant la revendication 6 ou 7, caractérisé en ce que ledit rail de guidage hélicoïdal (6) est obtenu à partir d'une bande ou d'un segment de plaque en forme de bande respectivement, enroulé en hélice et dont la largeur diminue progressivement de 6 cm environ à l'extrémité supérieure de la chambre (1) jusqu'à 2 cm environ à son extrémité inférieure.

9. Dispositif suivant l'une quelconque des revendications 6 à 8, caractérisé en ce que la paroi (2) de la chambre sensiblement cylindrique a une épaisseur de paroi relativement forte, afin d'emmagasiner l'énergie thermique fournie par le gaz chauffé.